# EUROPEAN PATENT APPLICATION

(11) **EP 4 732 903 A1**
(43) Date of publication of application: **29.04.2026**
(21) Application number: 24306815.2
(22) Date of filing: 28.10.2024
(51) Int. Cl.: A61P 25/00, C07D 211/06, A61K 31/4453, C07C 15/02

(54) **CO-CRYSTALS OF PITOLISANT HYDROCHLORIDE**

(71) Applicant: BIOPROJET Pharma, 75002 Paris (FR)
(72) Inventor: CAPET, Marc, 35520 MELESSE (FR); QUERNIARD, Florian, 76960 NOTRE-DAME DE BONDEVILLE (FR); PITON, Nelly, 35740 PACÉ (FR)
(74) Representative: Lavoix

(57) **Abstract**

The present disclosure relates generally to new co-crystals of pitolisant hydrochloride with coformers, pharmaceutical compositions comprising said co-crystals, dosage forms comprising said co-crystals, and methods of treating a disease or disorder with co-crystals, or with a pharmaceutical composition or dosage form comprising said co-crystals.

## Description

### BACKGROUND

Crystalline forms of an active pharmaceutical ingredient is an important consideration in the pharmaceutical industry. Different crystalline forms (polymorphs) of a drug molecule can present nonequivalent physicochemical properties and mechanical characteristics, due to varying factors such as crystal packing and molecular orientation, which can impact various physicochemical properties of the drug material, such as the hardness, hygroscopicity, solubility, stability, and pharmacokinetics.

Co-crystals represent another interesting and promising research area amongst pharmaceutical scientists to fine-tune the physicochemical properties of drug materials. Co-crystals can have significant implications in pharmaceuticals, affecting for example the solubility and dissolution profiles, as well as stability and bioavailability properties.

They may, in particular, affect the choice of excipients in a formulation, suitable routes of administration, dosage, the shelf-life, and also suitable methods for production and packaging.

Pitolisant is a selective antagonist or inverse agonist of the histamine H3 receptor used to treat type 1 or 2 narcolepsy. Pitolisant hydrochloride is a drug molecule that is useful for treating various diseases and disorders, particularly sleep disorders such as excessive daytime sleepiness (EDS) and cataplexy, that is marketed as WAKIX^{®}. WAKIX^{®} contains crystalline pitolisant hydrochloride.

A crystalline form of pitolisant hydrochloride referred to herein as Form I has been disclosed in U.S. Patent No. 8,207,197 that is incorporated herein by reference in its entirety.

An alternative crystalline form of pitolisant hydrochloride referred to as Form II is disclosed in U.S. Patent application No. 18/621,972 and PCT/FR2024/050398 that are also incorporated herein by reference in their entirety.

Pitolisant is typically absorbed with a peak plasma concentration reached approximately three hours after administration. In order to achieve its awakening effect without affecting the nocturnal sleep of the patient, it is desirable that the effect of pitolisant is triggered as soon as possible after its administration. There is thus a need to provide alternative forms to enhance e.g. the bioavailability by increasing the solubility and dissolution rate.

Co-crystals of pitolisant have never been investigated.

### SUMMARY

The present disclosure relates generally to co-crystals of pitolisant hydrochloride, pharmaceutical compositions and dosage forms comprising said co-crystals, as well as methods of treating a disease or disorder with said co-crystals or a pharmaceutical composition or dosage form comprising said co-crystals.

In a first object, the present disclosure relates to co-crystals of pitolisant hydrochloride represented by Formula (A): 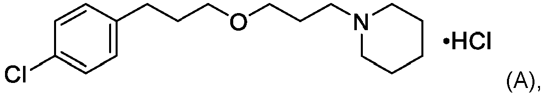 with a benzoic acid derivative, such as of formula (B):

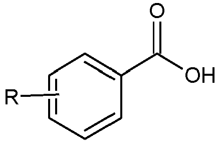

Where R is located on any available position on position 2, 3 or 4 of the phenyl ring with respect to the carboxy group, and R is chosen from the group consisting in NH₂, OH, Halogen atoms and CN.

In another object, the present disclosure relates to a pharmaceutical composition comprising a co-crystal disclosed herein, and optionally a pharmaceutically acceptable excipient.

In a further object, the present disclosure relates to a dosage form comprising said co-crystal, or pharmaceutical composition disclosed herein. The dosage form may be any suitable dosage form, such as a tablet, caplet, or capsule.

In a still further object, the present disclosure relates to a method of treating a disease or disorder, comprising administering to a subject in need thereof said co-crystal, pharmaceutical composition, or dosage form disclosed herein. The disease or disorder may be a sleep disorder. For example, the disease or disorder may be excessive daytime sleepiness (EDS), cataplexy, narcolepsy, sleep apnea (e.g., obstructive sleep apnea), sleep induced apnea, diurnal somnolence, severe fatigue or idiopathic hypersomnia. In some embodiments, the disease or disorder is excessive daytime sleepiness (EDS). In some embodiments, the disease or disorder is cataplexy. In a method disclosed herein, the subject to be treated may have narcolepsy, and/or may be an adult with narcolepsy.

In a still further object, the present disclosure relates to said co-crystal, pharmaceutical composition, or dosage form disclosed herein, for use in the treatment of a disease or disorder (*e.g.*, a disease or disorder disclosed herein), optionally, wherein the disease or disorder is excessive daytime sleepiness (EDS), cataplexy, narcolepsy, sleep apnea (*e.g.*, obstructive sleep apnea), sleep induced apnea, diurnal somnolence, severe fatigue or idiopathic hypersomnia. The disease or disorder may be in a subject with narcolepsy (*e.g.*, an adult subject with narcolepsy). In some aspects, the present disclosure relates to said co-crystal of pitolisant hydrochloride in the treatment of a disease or disorder (*e.g.*, a disease or disorder disclosed herein), optionally, wherein the disease or disorder is excessive daytime sleepiness (EDS), cataplexy, narcolepsy, sleep apnea (*e.g.*, obstructive sleep apnea), sleep induced apnea, or diurnal somnolence. The disease or disorder may be in a subject with narcolepsy (*e.g.*, an adult subject with narcolepsy).

In some aspects, the present disclosure relates to the use of said co-crystal, pharmaceutical composition, or dosage form disclosed herein, for the manufacture of a medicament for the treatment of a disease or disorder (*e.g.*, a disease or disorder disclosed herein), optionally, wherein the disease or disorder is excessive daytime sleepiness (EDS), cataplexy, narcolepsy, sleep apnea (*e.g.*, obstructive sleep apnea), sleep induced apnea, diurnal somnolence, severe fatigue or idiopathic hypersomnia. The disease or disorder can be in a subject with narcolepsy (*e.g.*, an adult subject with narcolepsy). In some aspects, the present disclosure relates to the use of said co-crystal of pitolisant hydrochloride for the manufacture of a medicament for the treatment of a disease or disorder (*e.g.*, a disease or disorder disclosed herein), optionally, wherein the disease or disorder is excessive daytime sleepiness (EDS), cataplexy, narcolepsy, sleep apnea (*e.g.*, obstructive sleep apnea), sleep induced apnea, diurnal somnolence, severe fatigue or idiopathic hypersomnia. The disease or disorder can be in a subject with narcolepsy (*e.g.*, an adult subject with narcolepsy).

In some aspects, the present disclosure relates to said co-crystal, pharmaceutical composition, or dosage form as disclosed above for use in treating a disease or disorder in a subject in need thereof, such as a disease or disorder disclosed herein, *e.g*., a sleep disorder, excessive daytime sleepiness (EDS), cataplexy, narcolepsy, sleep apnea (*e.g.*, obstructive sleep apnea), sleep induced apnea, diurnal somnolence, severe fatigue or idiopathic hypersomnia. For example, said co-crystal of pitolisant hydrochloride is for use in treating a disease or disorder in a subject in need thereof, such as a disease or disorder disclosed herein, e.g., a sleep disorder, excessive daytime sleepiness (EDS), cataplexy, narcolepsy, sleep apnea (*e.g.*, obstructive sleep apnea), sleep induced apnea, diurnal somnolence, severe fatigue or idiopathic hypersomnia. In some aspects, the disease or disorder is excessive daytime sleepiness (EDS). In some aspects, the disease or disorder is cataplexy. In some aspects, the subject has narcolepsy (*e.g.*, the subject is an adult with narcolepsy).

### DETAILED DESCRIPTION

### Definitions

The articles "a" and "an" are used herein to refer to one or more than one (*i.e.,* to at least one) of the grammatical object of the article. By way of example, "an element" means one element or more than one element.

The term "about" when referring to a measurable value such as an amount, a temporal duration, and the like, is meant to encompass variations of ±20% or less, or in some instances ±15% or less, or in some instances ±10% or less, or in some instances ±5% or less, or in some instances ±1% or less, or in some instances ±0.1% or less, from the specified value, as such variations are appropriate.

The phrase "and/or" as used herein should be understood to mean "either or both" of the elements so conjoined, *i.e.,* elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified. Thus, as a non-limiting example, a reference to "A and/or B," when used in conjunction with openended language such as "comprising" can refer, in one embodiment, to A only (optionally including elements other than B); in another embodiment, to B only (optionally including elements other than A); in yet another embodiment, to both A and B (optionally including other elements); etc.

The terms "administer," "administering," or "administration," as used herein refer to implanting, absorbing, ingesting, injecting, inhaling, or otherwise introducing a compound (*e.g., a co-crystal*), dosage form, or pharmaceutical composition.

The terms "comprise," "comprises," and "comprising" are used herein in a non-exclusive sense, except where the context requires otherwise. Likewise, the term "include" and its grammatical variants are intended to be non-limiting, such that recitation of items in a list is not to the exclusion of other like items that can be substituted or added to the listed items. The term "effective amount" or a "therapeutically effective amount" as used herein refers to an amount of a compound, a dosage form, or a pharmaceutical composition, described herein, which is sufficient to achieve a desired result under the conditions of administration. For example, an effective amount of a compound, dosage form, or pharmaceutical composition disclosed herein for treating excessive sleep disorder (EDS), *e.g.,* in a subject with narcolepsy, is an amount that can reduce the effects of the EDS, and/or reduce or eliminate the severity of a symptom associated with the EDS. A skilled clinician can determine appropriate dosing based on a variety of considerations including the severity of the disease, the subject's age, weight, general health and other considerations. A compound (*e.g.,* a co-crystal), dosage form, or pharmaceutical composition disclosed herein may be administered to provide an amount of about 0.01 mg to about 250 mg (e.g., about 0.1 mg to about 100 mg) of a pharmaceutically active agent, e.g., about 1 mg, about 2 mg, about 3 mg, about 4 mg, about 5 mg, about 10 mg, about 15 mg, about 20 mg, about 30 mg, or about 40 mg.

The term "pharmaceutically acceptable excipient" as used herein refers to a non-toxic material that may be formulated with a compound disclosed herein (*e.g.,* a co-crystal) to provide a pharmaceutical composition. Preferably, the pharmaceutically acceptable excipient is inert and does not interfere with the pharmacological activity of a compound which it is formulated with. Pharmaceutically acceptable excipients useful in the manufacture of the pharmaceutical compositions disclosed herein are any of those well known in the art, and include without limitation, diluents, dispersing agents, granulating agents, surface active agents, emulsifiers, disintegrating agents (sometimes referred to herein as disintegrants), binding agents (sometimes referred to herein as binders), preservatives, buffering agents (sometimes referred to herein as buffers), lubricating agents (sometimes referred to herein as lubricants), glidants, adjuvants, fillers, wetting agents, suspending agents, solvents, dispersion media, ion exchangers, salts, electrolytes, waxes, and/or oils, and the like.

For example, a pharmaceutically acceptable excipient may be alumina, a phosphate (*e.g.,* calcium phosphate, dicalcium phosphate, tricalcium phosphate, disodium hydrogen phosphate, potassium hydrogen phosphate), a sulfate (*e.g.,* calcium sulfate), a cellulose, kaolin, bentonite, lactose, mannitol, sorbitol, sucrose, inositol, compressible sugar, trehalose, xylitol, acacia, gelatin, glucose, maltodextrin, starch (*e.g.,* corn starch, potato starch), sodium starch glycolate, starch derivatives, an amino acid, magnesium carbonate, polyvinylpyrrolidone (PVP, povidone) (*e.g.,* crosslinked PVP, crospovidone), polyvinyl alcohol, tragacanth, polyethylene glycol, mineral clay powders, croscarmellose, poloxamer, fatty acids or salts thereof (*e.g.,* lauric acid, sodium lauryl sulfate, stearic acid, calcium stearate, magnesium stearate, aluminum stearate, oleic acid), hydrogenated vegetable oils, talc, titanium dioxide, glyceryl behenate, silicon dioxide (*e.g.,* colloidal silicon dioxide), a silicate salt (e.g., magnesium trisilicate), lecithin, serum protein (*e.g.,* human serum albumin), sorbic acid, potassium sorbate, a metal cation salt (*e.g.,* a sodium salt, such as sodium chloride, a potassium salt, such as potassium chloride, a magnesium salt, such as or magnesium chloride, a zinc salt, such as zinc chloride), water, dimethylacetamide, protamine sulfate, wool fat, ethylenediaminetetraacetic acid (EDTA), a cyclodextrin (*e.g.,* CAPTISOL^{®}), a polysorbate (*e.g.,* TWEEN^{®}, *e.g.,* TWEEN^{®} 20 or TWEEN^{®} 80), and combinations thereof.

The term "pharmaceutically acceptable salt" as used herein refers to salts of a compound prepared with relatively non-toxic acids or bases, depending on the particular substituents found on the respective compound. When compounds contain relatively basic functionalities, acid addition salts can be obtained by contacting the neutral form of such compounds with a sufficient amount of the desired acid, either neat or in a suitable solvent (*e.g.,* an inert solvent). Examples of pharmaceutically acceptable acid addition salts include those derived from inorganic acids like hydrochloric acid, hydrobromic acid, nitric acid, carbonic acid, monohydrogencarbonic acid, phosphoric acid, monohydrogenphosphoric acid, dihydrogenphosphoric acid, sulfuric acid, monohydrogensulfuric acid, hydriodic acid, or phosphorous acids and the like, as well as the salts derived from organic acids like acetic acid, propionic acid, isobutyric acid, maleic acid, malonic acid, benzoic acid, succinic acid, suberic acid, fumaric acid, lactic acid, mandelic acid, pamoic acid, phthalic acid, benzenesulfonic acid, p-toluenesulfonic acid, citric acid, tartaric acid, methanesulfonic acid, oxalic acid, and the like. Also included are salts of amino acids such as arginate and the like, and salts of organic acids like glucuronic or galacturonic acids and the like. Other pharmaceutically acceptable salts known to those of skill in the art are suitable for pharmaceutical compositions relating to the present disclosure.

The term "solvate" as used herein refers to forms of a compound that are associated with a solvent, usually by a solvolysis reaction. This physical association may include hydrogen bonding. Conventional solvents include water, methanol, ethanol, acetic acid, dimethyl sulfoxide (DMSO), tetrahydrofuran (THF), diethyl ether, and the like. Compounds of the present disclosure may be prepared, *e.g.,* in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolable solvates. Representative solvates include hydrates, ethanolates, and methanolates.

The term "hydrate" as used herein refers to a compound which is associated with water. Typically, the number of the water molecules contained in a hydrate of a compound is in a definite ratio to the number of the compound molecules in the hydrate. Therefore, a hydrate of a compound may be represented, for example, by the general formula R•xH₂O, wherein R is the compound and wherein x is a number greater than 0. A given compound may form more than one type of hydrate, including, *e.g.,* monohydrates (x is 1), lower hydrates (x is a number greater than 0 and smaller than 1, *e.g.,* hemihydrates (R•0.5H₂O)), and polyhydrates (x is a number greater than 1, *e.g.,* dihydrates (R•2H₂O) and hexahydrates (R•6H₂O)).

The term "subject" as used herein refers to any animal, such as any mammal, including but not limited to, humans, non-human primates, rodents, dogs, and the like. Non-human primates include chimpanzees, cynomolgus monkeys, spider monkeys, and macaques (*e.g.,* Rhesus). Rodents include mice, rats, woodchucks, ferrets, rabbits, and hamsters. Domestic and game animals include cows, horses, pigs, deers, bisons, buffalos, feline species (*e.g.,* domestic cat), canine species (*e.g.,* dog, fox, wolf), avian species, and fish. In some embodiments, the subject is a mammal (*e.g.,* a human, a rat, or a mouse). The subject can be male or female. The subject may be of any age, including an elderly human subject (*e.g.,* 65 years or older), a human subject that is not elderly (*e.g.,* less than 65 years old), or a human pediatric subject (*e.g.,* less than 18 years old). In preferred aspects, the subject is a human.

As used herein, the terms "treat," "treatment," "treating," or grammatically related terms, refer to a method of reducing the effects of a disease or disorder. As is readily appreciated in the art, full eradication of the disease, disorder, or symptoms thereof is preferred but not a requirement for treatment. Desirable effects of treatment include, but are not limited to, preventing occurrence or recurrence of the disease or disorder, alleviation of symptoms, diminishment of any direct or indirect pathological consequences of the disease or disorder, or other improvement of any sign, symptom, or consequence of the disease or disorder, such as prolonged survival, less morbidity, and/or a lessening of side effects.

Throughout this disclosure, various embodiments can be presented in a range format (*e.g.,* from X to Y). It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range. For example, description of a range, such as from 1 to 6, should be considered to have specifically disclosed subranges such as from 1 to 5, from 1 to 4, from 1 to 3, from 2 to 6, from 2 to 4, from 3 to 6, etc., as well as individual numbers within that range, e.g., 1, 2, 2.8, 3, 3.6, 4, 5, 5.4, and 6. As another example, a range such as 95-99% includes 95%, 96%, 97%, 98%, or 99% and all subranges such as 96-99%, 96-98%, 96-97%, 97-99%, 97-98%, etc. This applies regardless of the breadth of the range.

All publications (*e.g.,* scientific journal articles, patent publications, and the like) cited in this disclosure are incorporated by reference in their entirety. To the extent the material incorporated by reference contradicts or is inconsistent with this specification, the specification will supersede any such material. The citation of any references herein is not an admission that such references are prior art to the present disclosure. Various terms relating to aspects of the description are used throughout the specification and claims. Such terms are to be given their ordinary meaning in the art unless otherwise indicated. Other specifically defined terms are to be construed in a manner consistent with the definitions provided herein.

Compounds (*e.g.,* pharmaceutically active agents) disclosed herein may also comprise one or more isotopic substitutions. For example, hydrogen (H) may be in any isotopic form, including ¹H, ²H (D or deuterium), ³H (T or tritium); carbon (C) may be in any isotopic form, including ¹²C, ¹³C, and ¹⁴C; oxygen (O) may be in any isotopic form, including ¹⁶O and ¹⁸O; nitrogen (N) may be in any isotopic form, including ¹⁴N and ¹⁵N; and chlorine (CI) may be in any isotopic form, including ³⁵Cl and ³⁷Cl.

Various embodiments of the compounds, dosage forms, pharmaceutical compositions, and methods herein are described in further detail below, and additional definitions may be provided throughout the specification.

### Co-crystals of Pitolisant Hydrochloride

Pitolisant hydrochloride (which may also be referred to herein as pitolisant monohydrochloride) is also known as 1-[3-[3-(4-chlorophenyl)propoxy]propyl]-piperidine monohydrochloride. It may be represented by Formula (A): 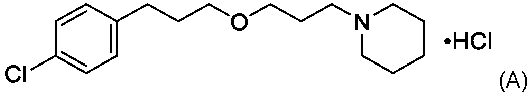 and may be referred to as "pitolisant HCl" herein.

It may be in the form of a solvate such as a hydrate, or in anhydrous form.

Disclosed herein are co-crystals of pitolisant hydrochloride, (ie) co-crystals comprising a compound represented by Formula (A): 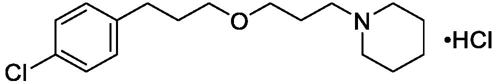 (A) and a coformer.

Co-crystals generally define a multicomponent system of active pharmaceutical ingredient (API) with a stoichiometric amount of a pharmaceutically acceptable coformer incorporated in the crystal lattice.

It will be understood that co-crystals are disclosed herein are crystalline, *i.e.,* not amorphous.

It will be also understood that the co-crystals represents a new crystalline entity, defining a new crystal repeating unit comprising pitolisant hydrochloride.

According to the invention, the co-crystals comprise a coformer in addition to pitolisant hydrochloride.

Additionally, the co-crystals may further comprise solvent, such as water. They may thus be qualified as solvates or hydrates. Typically, said solvate or water may be present in a stoichiometric amount.

The term "stoichiometric amount" refers to a defined respective ratio in number of an ingredient such as the coformer and/or the solvate or water, with respect to pitolisant hydrochloride.

The stoichiometry is considered for a given complete crystal repeating unit or the asymmetric unit.

The term "asymmetric unit" as used herein refers to the smallest portion of a crystal structure to which symmetry operations can be applied in order to generate the complete unit cell (the crystal repeating unit).

In an embodiment the invention relates to co-crystals of pitolisant hydrochloride and a coformer selected from benzoic acid derivatives, and the solvated or anhydrous forms of said co-crystal.

According to an embodiment, said coformer is a compound of formula (B):

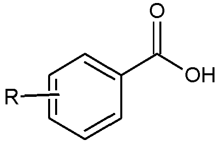

Where R is located on any available position on position 2, 3 or 4 of the phenyl ring with respect to the carboxyl group, and R is chosen from the group consisting of NH₂, OH, Halogen atoms and CN.

In an embodiment, in formula (B), R is located on the 4-position of the phenyl ring.

In an embodiment, in formula (B), R is chosen from NH₂ and OH.

In an embodiment, the compound of formula (B) is chosen from 4-aminobenzoic acid and 4-hydroxybenzoic acid.

Co-crystals can be characterized, and/or distinguished from other crystalline forms, using a suitable analytical technique such as X-ray powder diffraction (XRPD), differential scanning calorimetry (DSC) and/or thermogravimetric analysis (TGA).

For example, the co-crystals of the invention can be characterized using XRPD. The XRPD pattern can be obtained using any suitable protocol and with any suitable apparatus, such as a protocol or apparatus disclosed herein.

The XRPD used to characterize the co-crystals may be any suitable XRPD technique. For example, an XRPD pattern or peak disclosed herein can be acquired using Cu Kα radiation. For example, an XPRD technique disclosed herein can be used (see, e.g., Materials and Methods). The XRPD pattern may be obtained using a sample of between about 1 mg and about 10 mg (*e.g.,* between about 1 and about 5 mg, *e.g.,* about 2 mg, about 3 mg, about 4 mg, or about 5 mg).

XRPD can be used in particular to distinguish the co-crystals of the invention from the crystalline forms of pitolisant hydrochloride, such as its Form I and Form II.

For reference:
Form I of pitolisant hydrochloride typically shows a typical diffraction pattern as illustrated in U.S. Patent No. 8,207,197 and typically comprises peaks (2θ) at 11.2°, 19.9°, 20.7°, and 34.1° (± 0.2°), more particularly comprising peaks (2θ) at 11.2°, 15.4°, 16.3°, 16.9°, 17.8°, 19.9°, 20.7°, 21.0°, 21.8°, 22.6°, 24.5°, 24.6°, 25.0°, 25.5°, 26.3°, 28.3°, 30.3°, 34.1°, 35.8°, 40.0°, and 46.0° (± 0.2°).
Form II of pitolisant hydrochloride shows a typical diffraction pattern disclosed in U.S. Patent application No. 18/621,972 and PCT/FR2024/050398, and typically comprises comprising at least one of the following peaks, in terms of 2-theta (2θ), at 16.8°, 18.2°, 18.5°, 21.0°, and 25.1° (± 0.2°), particularly comprising at least one of the characteristic peaks (2θ) at 15.8°, 16.8°, 18.2°, 18.5°, 18.8°, 19.3°, 20.1°, 21.1°, and 25.1° (± 0.2°).

The co-crystals can also be characterized and/or distinguished from other crystalline forms by using DSC.

The DSC protocol used to determine an endothermic peak, a melting point, as disclosed herein, may be any suitable DSC protocol. For example, the DSC can be carried out using a sample of a few milligrams of the tested crystalline form, *e.g.*, between about 1 mg and about 10 mg, *e.g.,* between about 1 and about 5 mg, *e.g.,* about 2 mg, about 3 mg, about 4 mg, or about 5 mg.

The DSC may be carried out under nitrogen. The DSC may be carried out at a heating rate of about 10 °C/min. The DSC may be carried out within a temperature range between about 0 °C and about 300 °C, *e.g.*, between about 73 °C and about 150 °C.

For example, the DSC used to determine an endothermic peak or melting point disclosed herein can be carried out with one or more, or all of, the following steps: (i) heating from about 20 °C to about 150 °C at a rate of about 10 °C/min; (ii) cooling from about 150 °C to about 0 °C at a rate of 10 °C/min; (iii) heating from about 0 °C to about 140 °C at a rate of about 10 °C/min; (iv) cooling from about 140 °C to about 0 °C at a rate of about 200 °C/min; (v) holding at about 0 °C for about 2 minutes; (vi) heating from about 0 °C to about 140 °C at a rate of about 10 °C/min; (vii) cooling from about 140 °C to about 73 °C at a rate of 10 °C/min; (viii) holding at about 73 °C for about 4 minutes; and (ix) heating from about 73 °C to about 150 °C at a rate of about 10 °C/min.

DSC can be used in particular to distinguish the co-crystals of the invention from the crystalline forms of pitolisant hydrochloride, such as its Form I and Form II.

For reference:
Form I of pitolisant hydrochloride typically shows an endothermic peak with an onset between about 115 °C and about 119 °C, as obtained by DSC, e.g., between about 116 °C and about 118 °C, *e.g.,* about 117 °C; and
Form II of pitolisant hydrochloride typically shows an endothermic peak with an onset between about 91 °C and about 96 °C, *e.g.,* between about 92 °C and about 95 °C, or between about 93 °C and about 94 °C.

For example, the co-crystals of the invention can be characterized using thermogravimetry (TGA), in particular for identifying weight losses with regard to the temperature.

A representative illustration of the co-crystals of the invention is given in the following Examples and appended Figures.

According to an embodiment, the co-crystals comprise pitolisant HCl and 4-aminobenzoic acid, and optional water.

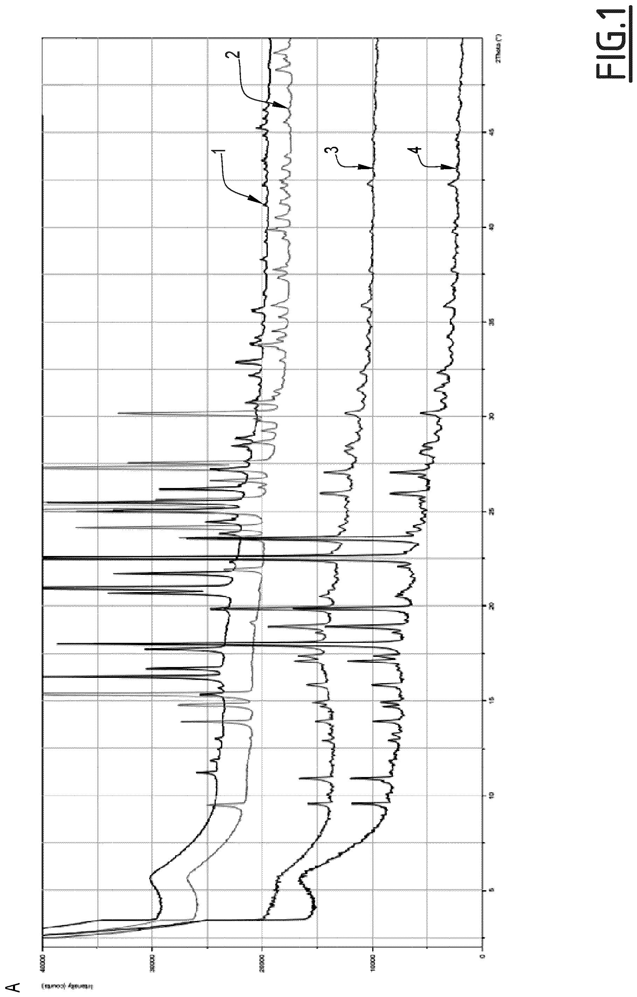

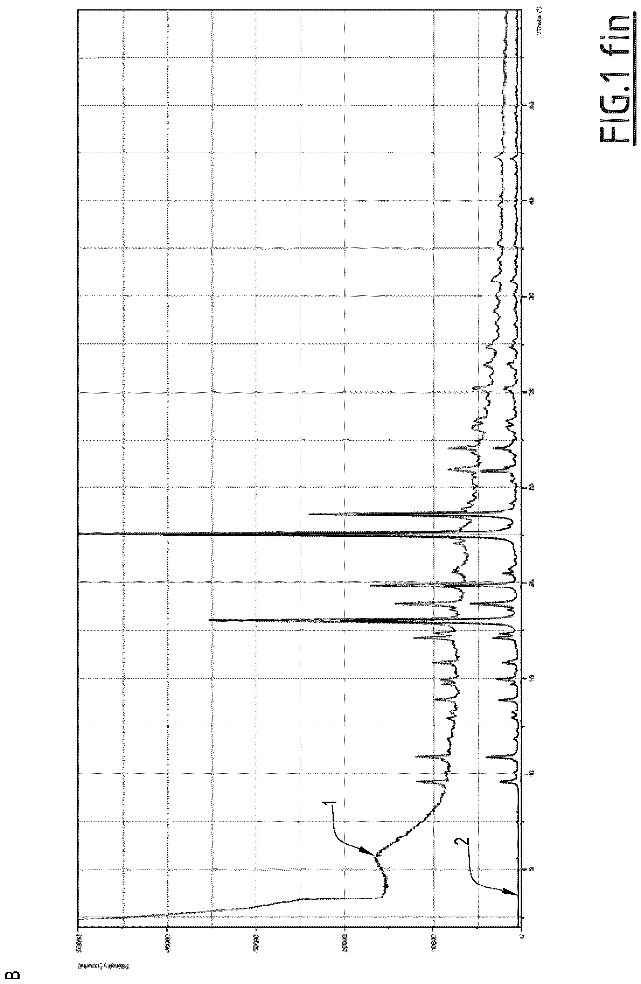

A representative example of a co-crystal comprising pitolisant HCl and 4-aminobenzoic acid (hereafter called co-crystal I) is illustrated in Figures 1, 4 and 6.

Typically, said co-crystal I may be characterized by having an X-ray diffraction pattern comprising one or more, or all of the following peaks, in terms of 2-theta (2θ), at 10.88°, 13.21°, 14.97°, 15.81°, 17.08°, 17.32°, 17.99°, 18.56°, 18.91°, 20.48°, 22.49°, 25.85° (± 0.2°).

Particularly, said co-crystal I has an X-ray diffraction pattern that comprises one or more, or all of the characteristic peaks (2θ) at 9.60°, 10.88°, 12.93°, 13.21°, 13.87°, 14.71°, 14.97°, 15.81°, 15.90° 17.08°, 17.32°, 17.99°, 18.56°, 18.91°, 19.85°, 20.48°, 22.49°, 23.02°, 23.56°, 25.85°, 26.02°, 27.04°, 28.14°, 28.40°, 28.48°°(± 0.2°).

As an illustration, said co-crystal I may be characterized by having an X-ray diffraction pattern substantially as shown in pattern 2 in FIG. 1B.

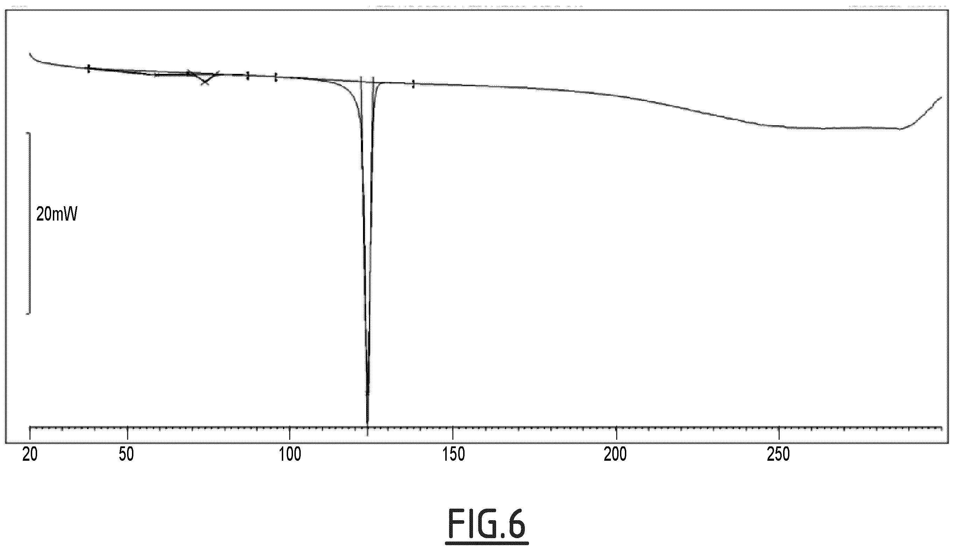

Additionally or alternatively, said co-crystal I may also be characterized by having a (DSC) thermogram substantially as shown in FIG. 6, and/or by having an endothermic peak substantially as shown illustrated in FIG.6.

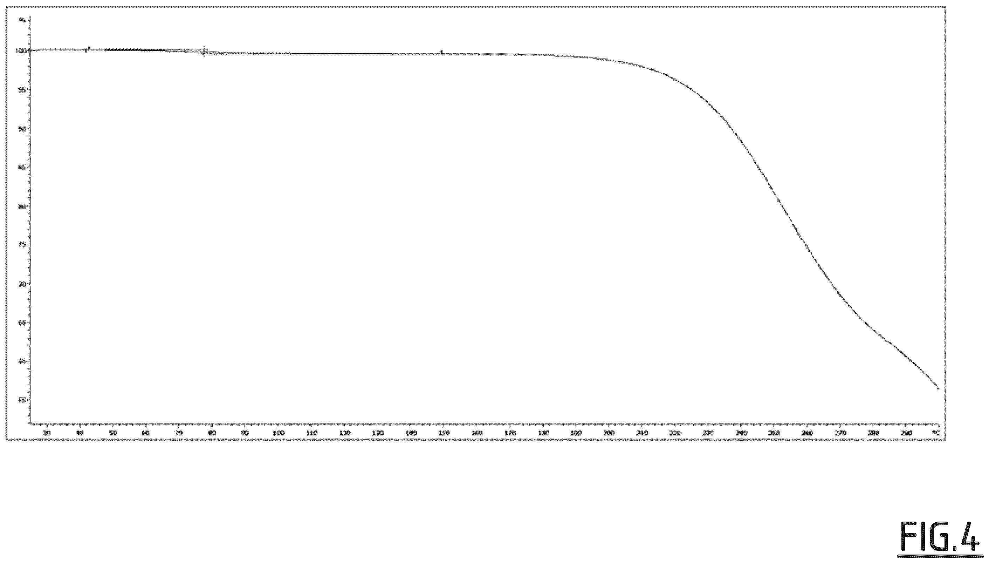

Additionally or alternatively, said co-crystal I may also be characterized by having a (TGA) thermogram substantially as shown in FIG. 4.

Typically, said co-crystal I has an asymmetric unit comprising three molecules of pitolisant HCl, six molecules of 4-aminobenzoic acid and half a molecule of water.

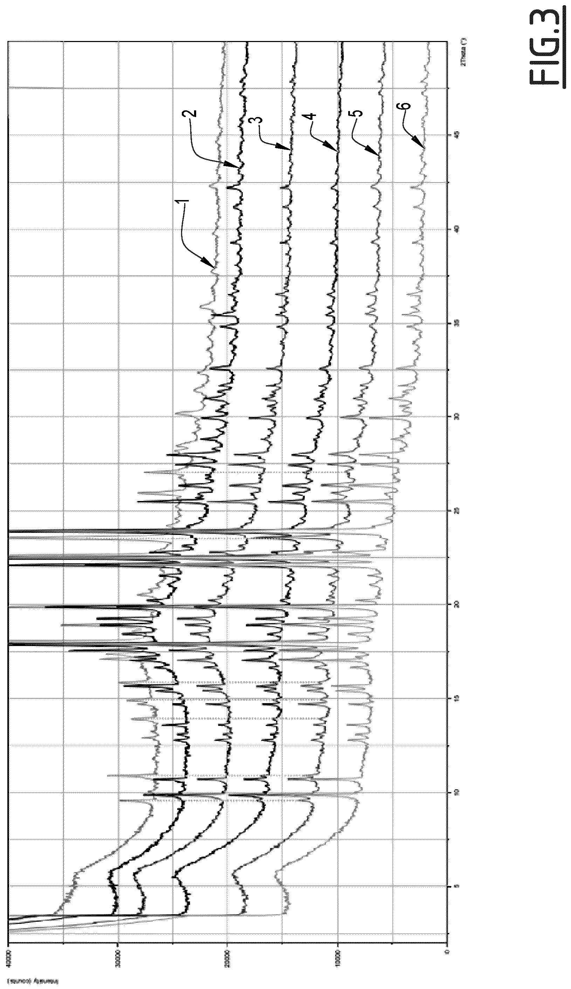

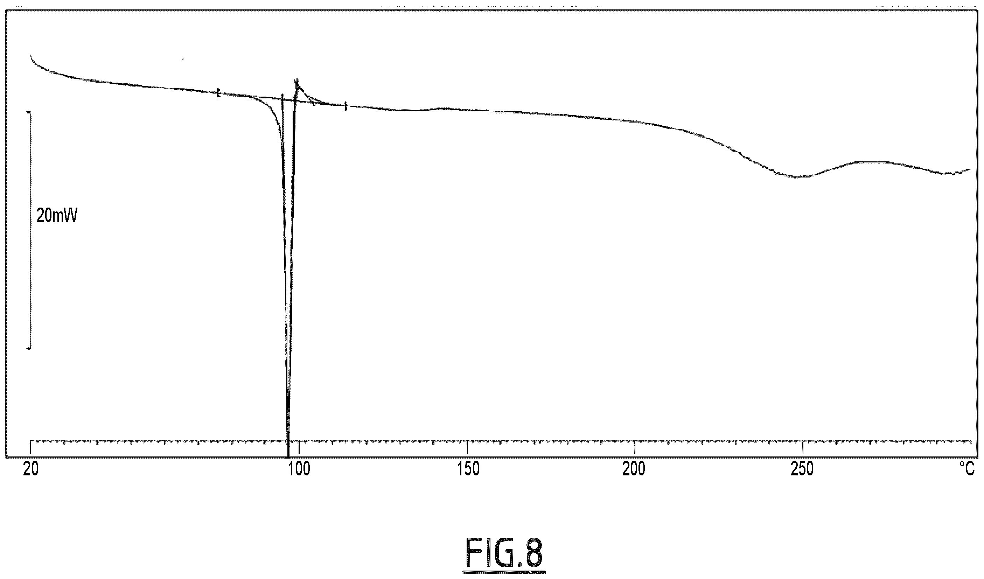

An alternative representative example of a co-crystal comprising pitolisant HCl and 4-aminobenzoic acid (hereafter called co-crystal III) is illustrated in Figures 3 and 8.

Typically, said co-crystal III may be characterized by having an X-ray diffraction pattern comprising one or more, or all of the following peaks, in terms of 2-theta (2θ), at 9.85°, 10.69°, 12.79°, 17.03°, 17.58°, 17.91°, 18.44°, 18.92°, 19.25°, 22.11°, 22.48°, 27.99° (± 0.2°).

Particularly, said co-crystal III has an X-ray diffraction pattern that comprises one or more, or all of the characteristic peaks (2θ) at 9.85°, 10.69°, 12.79°, 14.68°, 15.42°, 15.66°, 17.03°, 17.58°, 17.91°, 18.44°, 18.92°, 19.25°, 19.87°, 22.11°, 22.48°, 22.77°, 23.92°, 25.48°, 26.36°, 27.45°, 27.99°, 29.95° (± 0.2°).

As an illustration, said co-crystal III may be characterized by having an X-ray diffraction pattern substantially as shown in pattern 6 in FIG. 3.

Additionally or alternatively, said co-crystal III may also be characterized by having a (DSC) thermogram substantially as shown in FIG. 8, and/or by having an endothermic peak substantially as shown in FIG. 8.

According to a still alternative embodiment, the co-crystals may comprise pitolisant HCl and 4-hydroxybenzoic acid.

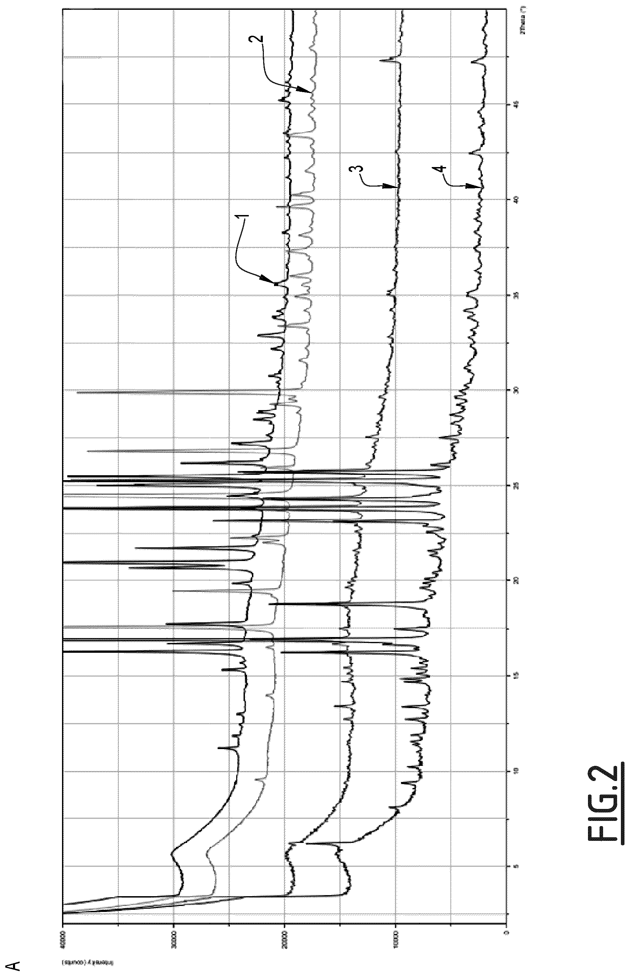

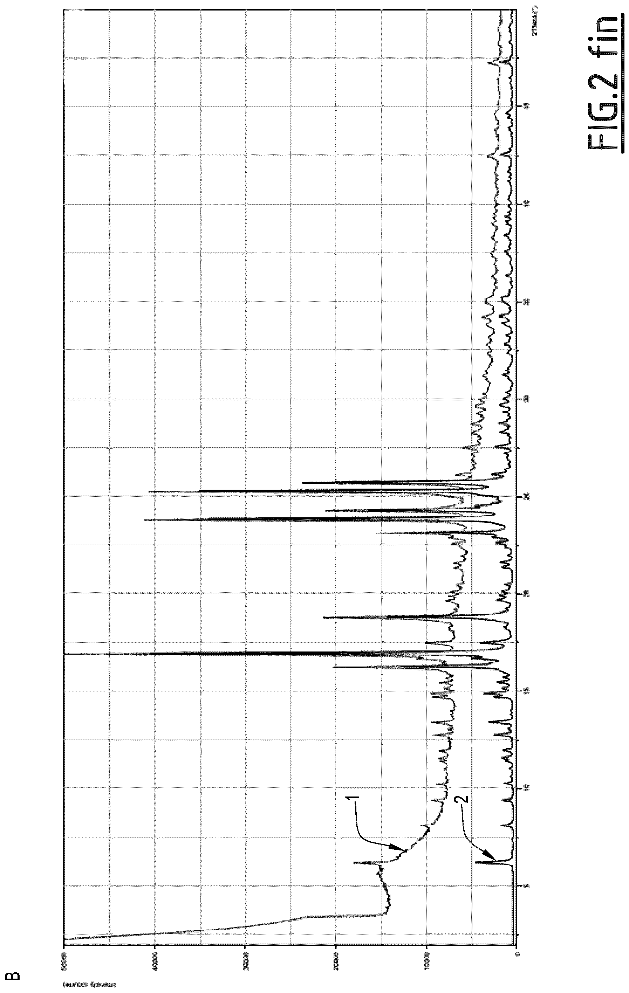

A representative example of a co-crystal comprising pitolisant HCl and 4-hydroxybenzoic acid (hereafter called co-crystal II) is illustrated in Figures 2, 5 and 7.

Typically, said co-crystal II may be characterized by having an X-ray diffraction pattern comprising one or more, or all of the following peaks, in terms of 2-theta (2θ), at 6.21°, 8.11°, 9.41°, 12.75°, 13.41°, 14.71°, 14.88°, 16.94°, 18.82°, 24.29°, 25.26°, 25.71° (± 0.2°). Particularly, said co-crystal II has an X-ray diffraction pattern that comprises one or more, or all of the characteristic peaks (2θ) at 6.21°, 8.11°, 9.41°, 12.75°, 13.41°, 14.71°, 14.88°, 16.26°, 16.68°, 16.94°, 17.48°, 18.82°, 22.91°, 23.13°, 23.82°, 24.29°, 24.49°, 24.61°, 25.26°, 25.71°, 26.13°, 27.55° (± 0.2°).

As an illustration, said co-crystal II may be characterized by having an X-ray diffraction pattern substantially as shown in pattern 2 in FIG. 2B.

Typically, said co-crystal II is anhydrous.

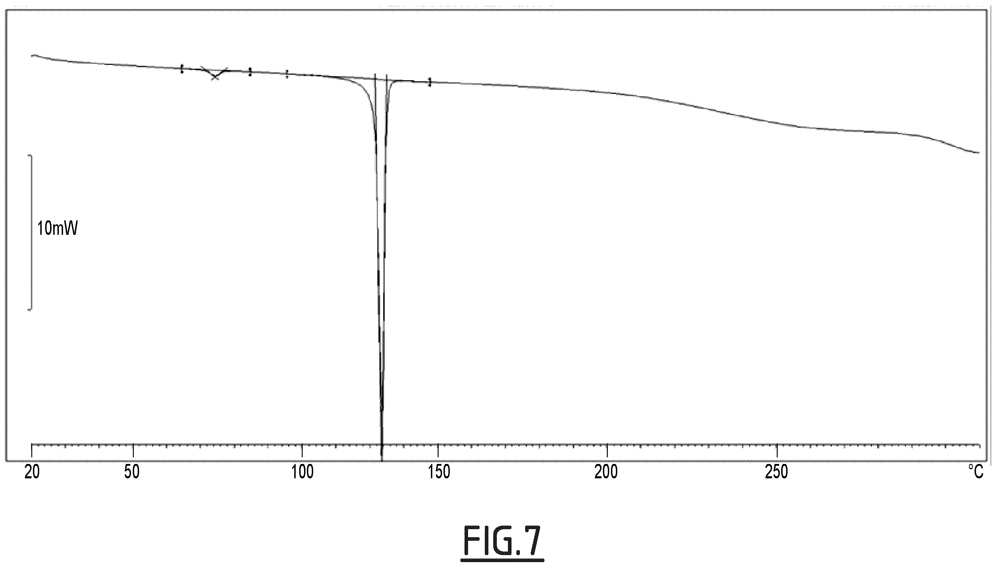

Additionally or alternatively, said co-crystal II may also be characterized by having a (DSC) thermogram substantially as shown in FIG. 7, and/or by having an endothermic peak substantially as shown in FIG. 7.

Additionally or alternatively, said co-crystal II may also be characterized by having a (TGA) thermogram substantially as shown in FIG. 7.

Typically, said co-crystal II has an asymmetric unit comprising two molecules of pitolisant HCl and four molecules of 4-hydroxybenzoic acid.

### Process of preparation of the co-crystals of the invention

Disclosed herein are processes of synthesis of the co-crystals of the invention.

Said process typically comprises mixing pitolisant hydrochloride and said coformer in an organic solvent.

According to an embodiment, said solvent may be chosen from aprotic solvents, typically polar aprotic such as ethyl acetate, and apolar aprotic solvents such as 1,4-dioxane. Preferably, when said coformer is 4-aminobenzoic acid, said organic solvent is an apolar aprotic solvent.

Preferably, when said coformer is 4-hydroxybenzoic acid, said organic solvent is a polar aprotic solvent.

Said mixing is typically conducted at a temperature comprised between 0°C and 45°C, preferably at room temperature.

The process may further comprise the additional step of filtering and optionally drying the resulting suspended product.

### Dosage Forms and Pharmaceutical Compositions

Disclosed herein are dosage forms and pharmaceutical compositions comprising a co-crystal of the invention, and optionally one or more pharmaceutically acceptable excipients. The pharmaceutically acceptable excipient may be any suitable pharmaceutically acceptable excipient, such as a pharmaceutically acceptable excipient disclosed herein. In some embodiments, the pharmaceutically acceptable excipient is selected from the group consisting of colloidal silicon dioxide, crospovidone, magnesium stearate, microcrystalline cellulose, polyethylene glycol, polyvinyl alcohol, talc, and titanium dioxide.

The dosage form or pharmaceutical composition can comprise a therapeutically effective amount of a co-crystal of the invention. For example, the dosage form or pharmaceutical composition may comprise between about 1 mg and about 200 mg of a co-crystal of the invention, *e.g.,* between about 1 mg and about 100 mg, between about 1 mg and about 50 mg, between about 10 mg and about 25 mg, or between about 1 mg and about 10 mg, of co-crystal, *e.g.,* about 3 mg, about 4 mg, about 5 mg, about 6 mg, about 8 mg, about 10 mg, about 12 mg, about 15 mg, about 20 mg, about 25 mg, about 30 mg, about 40 mg, or about 50 mg, of a co-crystal of the invention.

It will be understood that because a co-crystal of the invention comprises a pharmaceutically acceptable salt of pitolisant and a coformer and optionally a solvate, the amount of pharmaceutically active agent in the dosage form or pharmaceutical composition will be slightly higher than the equivalent amount of free base. The dosage forms of the present disclosure can be tablets, caplets, capsules, suspensions, granules, powders, or the like. The dosage forms or pharmaceutical compositions of the present disclosure can further comprise one or more pharmaceutically acceptable excipients, such as diluents, dispersing agents, granulating agents, surface active agents, emulsifiers, disintegrating agents (sometimes referred to herein as disintegrants), binding agents (sometimes referred to herein as binders), preservatives, buffering agents, lubricating agents (sometimes referred to herein as lubricants), glidants, adjuvants, fillers, wetting agents, suspending agents, solvents, dispersion media, ion exchangers, salts, electrolytes, waxes, and/or oils, and the like. The pharmaceutical composition or dosage form may comprise a pharmaceutically acceptable excipient disclosed herein, or a combination of pharmaceutically acceptable excipients disclosed herein. For example, a dosage form or pharmaceutical composition of the present disclosure may comprise one or more, or all of, the following pharmaceutically acceptable excipients: colloidal silicon dioxide, crospovidone, magnesium stearate, microcrystalline cellulose, polyethylene glycol, polyvinyl alcohol, talc, and titanium dioxide. Each pharmaceutically acceptable excipient can be present in the dosage form or pharmaceutical composition in any suitable amount. For example, a pharmaceutically acceptable excipient can be present in the dosage form or pharmaceutical composition in an amount of between about 0.01% and about 95% by weight of the dosage form or pharmaceutical composition, e.g., between about 0.1% and about 25%, between about 1% and about 10%, between about 15% and about 95%, between about 0.01% and about 2%, by weight of the dosage form or pharmaceutical composition.

Dosage forms and pharmaceutical compositions of the present disclosure may be administered orally, parenterally, by inhalation, topically, rectally, nasally, buccally, vaginally, or by implantation.

Preparation of the dosage forms or pharmaceutical composition of the present disclosure can include conventional methods, such as blending, filling, compressing (*e.g.,* direct compression, compression of dry, wet or sintered granules), coating (*e.g.,* coating in a spray process), extrusion, granulation *(e.g.,* wet or dry granulation), pelleting *(e.g.,* direct pelleting), binding, powder layering (*e.g.,* onto active ingredient-free beads, or neutral cores or particles of pharmaceutically active agent), and rounding off.

### Methods of Treatment

The present disclosure further relates to a method for the treatment of a disease or disorder, comprising administering a co-crystal of the invention, or a dosage form or pharmaceutical composition disclosed herein, to a subject in need thereof.

The disease or disorder may be a sleep disorder (*e.g.*, excessive daytime sleepiness (EDS), cataplexy, narcolepsy, sleep apnea (e.g., obstructive sleep apnea), sleep induced apnea, diurnal somnolence, severe fatigue or idiopathic hypersomnia), central nervous system disorder (*e.g.,* epilepsy, Alzheimer's disease, Parkinson's disease, dementia (*e.g.,* dementia with Lewy bodies and/or vascular dementia), attention disorders, wakefulness disorders, memorization disorders, cognitive deficits (*e.g.*, in aged persons), psychiatric pathologies, depressive and asthenic states, vertigo, and motion sickness), obesity, psychosomatic disorders, respiratory disorders, allergic conditions, inflammatory conditions, cardiac conditions, gastrointestinal conditions, conditions of the urogenital system, conditions of the cutaneous system, stress, migraine, headache, pain, psychotropic disorders, asthma, bronchitis, rhinitis, tracheitis, gastric ulcers, duodenal ulcers, ulcerative colitis, Crohn's disease, irritable bowel syndrome (IBS), cystitis, metritis, urinary incontinence, fecal incontinence, urticaria, itching, arthritis, conjunctivitis, premenstrual syndrome, prostatic inflammations, genital disorders, rheumatic conditions, ocular conditions, sialorrhea, convulsion, depression, disorders of the hypothalamo-hypophyseal system, disorders of the cerebral circulation, and disorders of the immune system.

In preferred aspects, the disease or disorder is a sleep disorder. For example, the disease or disorder can be excessive daytime sleepiness (EDS) or cataplexy. The disease or disorder can be in subjects (*e.g.*, adult subjects) with narcolepsy.

The present disclosure further relates to a method for the prevention of undesirable side effects associated with using antipsychotic or antidepressant agents (*e.g.*, aripiprazole, clozapine, olanzapine, risperidone, quetiapine, sertindole, mirtazapine, amitryptiline, and paroxetine), comprising administering a co-crystal of the invention, or a dosage form or pharmaceutical composition of the present disclosure, to a subject in need thereof. Non-limiting examples of undesirable side effects associated with using antipsychotic or antidepressant agents includes weight gain, somnolence, and cognitive impairment.

The present disclosure further relates to a method for (i) inducing an extended state of wakefulness; (ii) improving cognitive processes; (iii) reducing food intake; and/or (iv) normalizing vestibular reflexes, comprising administering a co-crystal, or a dosage form or pharmaceutical composition disclosed herein, to a subject in need thereof.

A co-crystal of the invention, or a dosage form or pharmaceutical composition disclosed herein, may be administered once daily, twice daily, or more often. More than one dosage form can be administered at once to achieve a desired dose. A co-crystal of the invention, or a dosage form or pharmaceutical composition disclosed herein, may be taken with a frequency and in such an amount so that the total amount of pitolisant (in terms of free base) administered is within the range of from about 10 mg to about 50 mg per day, *e.g.,* about 15 mg to about 40 mg per day. A co-crystal of the invention, or a dosage form or pharmaceutical compositions disclosed herein, may be taken with a frequency and in such an amount so that the total amount of pitolisant (in terms of free base) administered is within the range of from about 17.8 mg to about 35.6 mg per day. For example, a subject may be administered orally two dosage forms once daily, where each dosage form comprises 4.45 mg pitolisant (in terms of free base), to achieve a daily dose of 8.9 mg pitolisant (in terms of free base). A subject may be administered orally one dosage form once daily, where the dosage form comprises 17.8 mg pitolisant (in terms of free base), to achieve a daily dose of 17.8 mg pitolisant (in terms of free base). A subject may be administered orally two dosage forms once daily, where each dosage form comprises 17.8 mg pitolisant (in terms of free base), to achieve a daily dose of 35.6 mg pitolisant (free base).

### FIGURES

Figure 1 are graphical representations and overlays of X-ray powder diffraction (XRPD) patterns relative to Example 1A (Co-crystal I).
Figure 1A illustrates the comparison of diffraction patterns of Pitolisant HCl (1), 4-aminobenzoic acid (2), Reference Co-crystal I (3), Prepared Co-crystal I (4), where "Reference Co-crystal I" refers to the Co-crystal I obtained during a co-crystal screening and "Prepared Co-crystal I" refers to Co-crystal I prepared and scaled-up using optimized conditions.
Figure 1B illustrates the comparison of the diffraction pattern of Co-crystal I (1) with the simulated pattern (2)
Figure 2 are graphical representations and overlays of X-ray powder diffraction (XRPD) patterns relative to Example 1B (Co-crystal II).
Figure 2A illustrates the comparison of diffraction patterns of Pitolisant HCl (1), 4-aminobenzoic acid (2), Reference Co-crystal II (3), Prepared Co-crystal II (4), where "Reference Co-crystal II" refers to the Co-crystal II obtained during a co-crystal screening and "Prepared Co-crystal II" refers to Co-crystal II prepared and scaled-up using optimized conditions.
Figure 2B illustrates the comparison of the diffraction pattern of Co-crystal II (1) with the simulated pattern (2)
Figure 3 is a graphical representation and overlay of X-ray powder diffraction (XRPD) patterns relative to Example 1C (Co-crystal III). It illustrates the comparison of diffraction patterns of Reference Co-crystal I (1), Reference Co-crystal III (2), Prepared Co-crystal III before filtration (3), 24hrs under N2 (nitrogen generator) (4), after 10 days under N2 (nitrogen generator) (5), one more day under N2 (gas cylinder) (6).
Figure 4 is a graphical representation of the thermogravimetric analysis (TGA) of the prepared Co-crystal I.
Figure 5 is a graphical representation of the TGA of the prepared Co-crystal II.
Figure 6 is a graphical representation of the Differential Scanning Calorimetry (DSC) curves of the prepared Co-crystal I.
Figure 7 is a graphical representation of the Differential Scanning Calorimetry (DSC) curves of the prepared Co-crystal III.
Figure 8 is a graphical representation of the superimposed Differential Scanning Calorimetry (DSC) curves of the prepared Co-crystal II.

### EXAMPLES

### Materials and Methods

### Reagents used include :

| Designation | Supplier |
|---|---|
| 4-Aminobenzoic acid | Sigma-Aldrich |
| 4-Hydroxybenzoic Acid | TCI Europe |
| 1.4-Dioxane | Carlo Erba |
| Ethyl acetate | Carlo Erba |

For this study, the following analytical means are used:
- X-ray powder diffraction (XRPD): for the characterization and identification of solid phases
- Differential Scanning Calorimetry (DSC): for the characterization of the thermal profile with regard to the temperature
- Thermogravimetry (TGA): for the characterization of weight losses with regard to the temperature
- Dynamic vapor sorption (DVS): for the measurement of water intake / outtake and the characterization of hydration / dehydration.
- Infrared spectroscopy (FTIR): for the characterization and identification of solid phases.
- Nuclear Magnetic Resonance (¹H-NMR): for the chemical characterization of solid phases
- High Performance Liquid Chromatography (HPLC): for the measurement of purity.
- Chloride titration: for characterization of chloride stoichiometry

### X-Ray Powder Diffraction (XRPD)

Analyses are performed in transmission mode (the sample is placed between Kapton^{®} and Polypropylene foils), on the angular range 2θ = 2 - 50°, with a step size of 0.026° and a time per step of 20.4s, unless stated otherwise. It is worth noting that Kapton^{®} exhibits a low intensity signal in the shape of a large hump around 2θ = 5.5°.

### Thermogravimetry (TGA)

Analyses are performed on a few milligrams of sample, in 100 µL sealed aluminum pans, punctured before analysis, under nitrogen flush at 50 mL/min. A temperature range between 25 °C and 300 °C is scanned at a 10 °C/min rate (unless otherwise stated).

### Differential Scanning Calorimetry (DSC)

Analyses are performed on a few milligrams of sample, in 40 µL sealed aluminum pans, punctured before analysis, under nitrogen flush at 50 mL/min. A temperature range between 20 °C and 300 °C is scanned at a 10 °C/min rate (unless otherwise stated).

### Dynamic Vapor Sorption (DVS)

Analyses are performed on a few milligrams, in open aluminum pans at 25 °C. The stability criterion for each step is a weight change lower than 0.002% on a 5 min time frame. The time criterion for each step is 100 min (minimum duration by step: 10 min). Relative humidity is scanned between 0%RH and 95%RH with 10%RH steps (40 - 0 - 95 - 0 - 95 ; unless otherwise stated).

### Infrared spectroscopy (FTIR)

Analyses are performed in ATR mode from 4000 cm⁻¹ to 525 cm⁻¹ (resolution of 4 cm⁻¹), with 32 scans for background and measurement.

### ¹H-NMR

The ¹H-NMR analyses are performed on a Brüker AVANCE III 300 MHz NMR spectrometer equipped with a Brüker BBFO probe at 25 °C (298 K). Samples are dissolved in DMSO-d₆ and 16 scans are recorded for each analysis.

### High Performance Liquid Chromatography (HPLC)

The analyses are performed on an Agilent Technologies 1260 Infinity II. The following method was used:
- Column: XSelect HSS T3 100Å, 3.5 µm, 4.6 mm x 100 mm
- Flow rate: 1 mL / min
- Temperature: 40 °C
- Detection: UV @ 220 nm
- Injection volume: 5 µL
- Sample preparation: ~1g/L in water
- Mobile phase A: water/0.1% formic acid
- Mobile phase B: acetonitrile/0.1% formic acid
- Duration: 16 min
- Needle wash : water

### HPLC gradient:

| Time | A | B |
|---|---|---|
| min | % | % |
| 0 | 95 | 5 |

| | | |
|---|---|---|
| 9.2 | 5 | 95 |
| 9.21 | 95 | 5 |
| 16 | 95 | 5 |

### Chloride titration

Chloride titration is performed on a Mettler Toledo Titration Excellence T50 equipped with a DMi-141-SC electrode. Silver nitrate 0.02 M is used as titrant in a 5 mL automatic burette. Measures are done in triplicate.

### X-Ray Diffractometer

Type : Panalytical Empyrean S3
Serial Number : DY2626
Incident Beam (Transmission Mode, Focusing mirror):
   Radius (mm): 240.0
   X-ray tube
      Name: Empyrean Cu LFF HR (9430 033 7310x) DK439187
      Anode Material: Cu (1.54 Å)
      Voltage (kV): 40.0
      Current (mA): 40.0
      Focus type: Line (Length (mm): 12.0 width (mm): 0.4 Take-off angle (°): 7.6)
   X-ray mirror
      Name: Focusing X-ray mirror for Cu radiation
      Crystal (W/Si Graded Elliptic)
      Acceptance angle (°): 0.800
      Length (mm): 55.3
   Soller slit
      Soller 0.04 rad.
      Opening (rad.): 0.04
   Anti-scatter slit:
      Fixed slit 1/2
      Type: Fixed
      Height (mm): 0.76
   Divergence slit
      Fixed slit 1/2°
      Distance to sample (mm): 180
      Type: Fixed
      Height (mm): 0.76
      Angle (°): 0.7209

### Diffracted Beam

Radius (mm): 240.0
Anti-scatter slit
   Name: AS slit 7.5 mm (PlXcel)
   type: Fixed
   Height (mm): 7.50
Soller slit
   Name: Large Soller slits 0.04 rad.
   Opening (rad.): 0.04
Detector
   Name: PIXcel1D-Medipix3 detector
   Type: RTMS detector
   PHD - Lower level (%): 25.0
   PHD - Upper level (%): 70.0
   Mode: Scanning
   Active length (°): 3.3482

### Thermogravimetric Analyzer

Type : Mettler Toledo TGA / DSC3+
Serial Number: B736632981
Purge Gas : Nitrogen (50 mL/min)

Pitolisant monohydrochloride can be prepared according to methods described in U.S. Patent No. 8,207,197, *e.g.*, following the protocol provided below.

Sodium 3-piperidinopropanolate (2.13 kg; 12.88 mol), 3-(4-chlorophenyl)propyl mesylate (1.12 kg; 4.51 mol), and 0.322 mol of 15-crown-5 in dry toluene (4.5 kg) were refluxed for 4 hours. The solvent was evaporated and the residue purified by column chromatography on silica gel (eluent: methylene chloride/methanol, 9:1). The obtained oil was distilled in fractionating equipment at reduced pressure (0.3-0.7 mmHg) and with a heating jacket at 207-210 °C. The head fractions and the distilled fractions were collected at 0.001-0.010 mmHg with a jacket temperature of 180-200° C, to provide pitolisant free base (1-[3-[3-(4-chlorophenyl)propoxy]propyl]-piperidine) as an oil (1.0 kg; 3.38 mol).

Distilled 1-[3-[3-(4-chlorophenyl)propoxy]propyl]-piperidine (1.0 kg) and anhydrous ethyl acetate (4.5 kg) were transferred to a 10-L glass vessel fitted with a cooling bath and a gas inlet. A stream of gaseous hydrogen chloride was bubbled in the reaction mixture at 20-25 °C. The pH of the solution was checked by taking a 0.5 mL sample of the reaction mixture and diluting it with 5 mL of deionized water, to obtain a pH of about 3-4.

The mixture was cooled to between -10° C and -12° C and stirred for 1 h. The precipitate was filtered by using a sintered glass filter and washed with 0.5 L of anhydrous ethyl acetate cooled to 0-5° C. The product was dried in a vacuum oven at 50 °C for a minimum period of 12 hours, to provide crude pitolisant monohydrochloride (1-[3-[3-(4-chlorophenyl)propoxy]propyl]-piperidine monohydrochloride) (1.10 kg).

A mixture of the crude pitolisant monohydrochloride, anhydrous ethyl acetate (3.98 kg) and isopropanol (0.35 kg) were heated slowly at 55-60° C in a 10 L glass vessel fitted with a heating and cooling system, and the resulting solution was filtered through a heat-isolated sintered glass filter, keeping the temperature at 55-60° C. The solution was transferred into a 10 L glass vessel and the mass was slowly cooled to 0-5° C for about 1 hour. The mixture was stirred at this temperature for 1 hour and the precipitate was filtered through a sintered glass filter. The solid was washed with a mixture of anhydrous ethyl acetate (1.6 kg) and isopropanol (0.14 kg) cooled at 0-5° C. The solid was dried in a vacuum oven at 50 °C for a minimum period of 12 hours to provide pure pitolisant monohydrochloride (1-[3-[3-(4-chlorophenyl)propoxy]propyl]-piperidine monohydrochloride) (M.p. 117-119° C; yield 80%; IR spectrum (KBr): bands at 1112 and 1101 (C-O Ether/St. asym), 2936 and 2868 (Alkane CH(CH₂))/St.), 1455 (Alkane CH(CH₂))/Deform.), 2647 and 2551 (Amine Salt/St.), 1492 (Amine/St.), 802 (Aromatic/Deform.) cm⁻¹.

A solution of the pure pitolisant monohydrochloride in acetone may be evaporated and dried at 70 °C for 17 hours, to provide crystals of Form I for use in DSC analyses.

### Example 1. Co-crystal preparations and characterization

### Example 1A : Co-crystal I

### Preparation

440.18 mg (1.32 mmol) of Pitolisant HCl, BP2.649, and 310.71 mg (2.27 mmol) of 4-aminobenzoic acid were suspended in 0.75 mL of 1,4-dioxane at room temperature. After stirring overnight at room temperature, the suspension was filtered on porosity-4 glass filter and dried under free air for 5 days. 324.75 mg of dry solid were collected (Co-Crystal I).

### Characterization

The diffraction pattern, the thermogravimetric analysis curve and the DSC curve of prepared Co-crystal I are given in Figures 1, 4 and 6 respectively.

The thermogravimetric analyses performed on the prepared Co-crystal I is plotted in Figure 4. It shows an important weight loss above 200 °C, probably corresponding to degradation. The weight loss over the range 30-145 °C is 0.62% for Co-crystal I. It must be related with the additional small endotherm spread from 40 to 70 °C visible in DSC.

The DSC curve obtained after the analyses of the prepared Co-crystal I is plotted in Figure 6. It shows a small endotherm spread from 40 to 70 °C, a tiny endothermic signal (2-5 J/g) at ca. 70 °C and a stronger endothermic event (94-96 J/g) at 121-122 °C.

### Example 1B : Co-crystal II

### Preparation

440.45 mg (1.32 mmol) of Pitolisant, BP2.649 and 309.44 mg (2.24 mmol) of 4-hydroxybenzoic acid were suspended in 0.75 mL of ethyl acetate at room temperature. After stirring overnight at room temperature, the suspension was filtered on porosity-4 glass filter and dried under free air for 5 days. 179.52 mg of dry solid were collected (sample 7B, co-crystal II).

### Characterization

The diffraction pattern, the TGA and DSC curves of prepared Co-crystal II are given in Figures 2, 5 and 8 respectively.

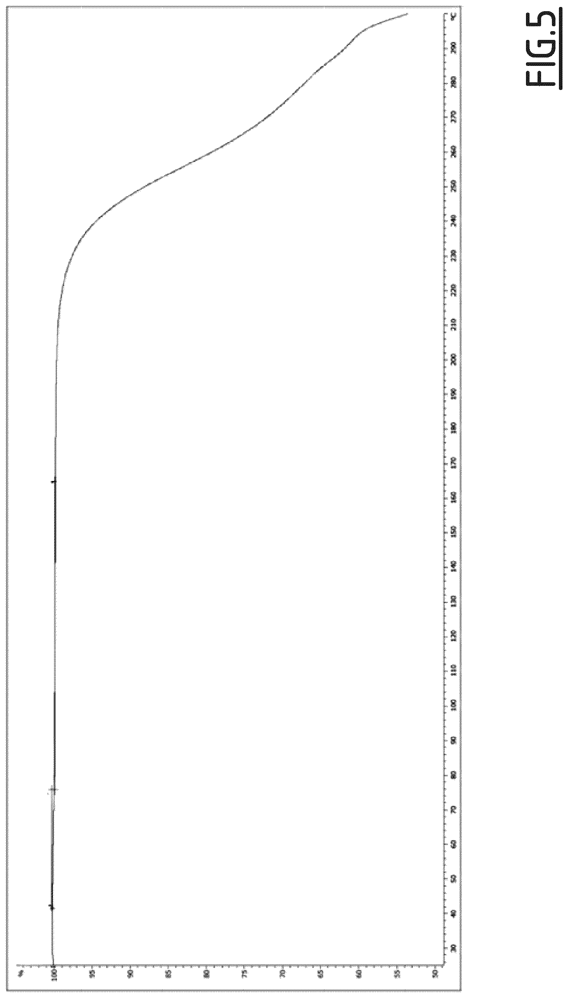

The thermogravimetric analysis performed on the Co-crystal II shows a tiny weight loss (0.3%) over the temperature range 25-170 °C (see Figure 5). Important weight loss, probably corresponding to degradation, occurs above 200 °C and continues beyond the range of the analysis.

The DSC curve obtained after the analysis of the Co-crystal II shows an endothermic event at 94.8 °C, that could correspond to the melting of the sample (see Figure 7). It is immediately followed by a small exotherm. Broad endotherms above 200 °C could correspond to degradation.

### Example 1C: Co-crystal III

### Preparation

440.21 mg (1.32 mmol) of Pitolisant, BP2.649 and 309.05 mg (2.27 mmol) of 4-aminobenzoic acid were suspended in 0.75 mL of 1,4-dioxane at room temperature. After stirring overnight at room temperature, the suspension was filtered on porosity-4 glass filter and dried under a nitrogen stream. 428 mg of wet solid were collected, labelled Co-crystal III and stored under a nitrogen stream in order to keep the Co-crystal III for 10 days. (see Figure 3).

The DSC curve obtained after the analyses of the prepared Co-crystal III obtained after drying is plotted in Figure 8. It shows a tiny endothermic signal (2-5 J/g) at ca. 70 °C and a stronger endothermic event (94-96 J/g) at 121-122 °C.

### Conclusions:

¹H-NMR and HPLC analyses of prepared Co-crystal I and Co-crystal III are compatible with a Pitolisant.HCl:4-aminobenzoic acid 1:2 co-crystal. Indeed, 1H-NMR shows 1.9 equivalent of 4-aminobenzoic acid while HPLC shows 1.8 equivalent. The solid has been shown to contain 5.9% of chloride, in agreement with the theoretical chloride content of 5.84% for a Pitolisant.HCl:4-aminobenzoic acid 1:2 co-crystal.

DVS analyses of Co-crystal I and Co-crystal III both show one hysteresis between 20 and 60%RH corresponding to about 0.5-0.6%wt of water (1/5 or 1/6 equivalent). Co-crystal III is obtained after the drying step (0%RH) of Co-crystal I, while Co-crystal I is obtained after the final step at 90%RH of Co-crystal III, with a final uptake of 1.9% (1/2 equivalent of water). TGA analyses of Co-crystals I and III show a mass loss of 0.6%wt for the former and 0.1%wt for the latter. It can be assumed that Co-crystal III is the anhydrous 1:2 co-crystal and that Co-crystal I is a hydrate of the 1:2 co-crystal. This hydrate could be non-stoichiometric: from 1/5 or 1/6 equivalent of water in the range 20-60%RH to one half equivalent or more at 90%RH or above.

¹H-NMR and HPLC analyses of prepared Co-crystal II are compatible with a Pitolisant.HCl:4-hydroxybenzoic acid 1:2 co-crystal. Indeed, 1H-NMR shows 1.9 equivalent of 4-hydroxybenzoic acid while HPLC shows 1.7 equivalent. The solid has been shown to contain 5.9% of chloride, in agreement with the theoretical chloride content of 5.83% for a Pitolisant.HCl:4-hydroxybenzoic acid 1:2 co-crystal.

DVS analysis only shows reversible sorption and tiny mass loss is observed in TGA analysis. Co-crystal II must be an anhydrous Pitolisant.HCl:4-hydroxybenzoic acid 1:2 co-crystal.

### Example 2 : Structure determination

### Samples :

102.42 mg of Co-crystal I and 58.57 mg of Co-crystal II were provided as is to synchrotron SOLEIL for X-ray measurements.

### Measurement methodology on PROXIMA-2A beamline

The experiment was performed on the beamline Proxima-2A. The sample were mounted on a loop and hold in place with vacuum grease. The measurement was performed at room temperature. The energy was set to 17 keV (λ= 0.72932 Å). A total of 3600 images with an angular step of 0.1° were measured. The detector EIGER X 9M was set at a distance of 120 mm from the sample. The resolution range is 14.22 - 0.99 Å. For each sample, one crystal was mounted then measured. The data processing, including the indexation and integration of the reflection data, was performed using Xia2 with Dials (Winter, G. 2010, Journal of Applied Crystallography 43, 186-190).

All crystals were weakly diffracting, especially at low resolution. Moreover, due to X-ray radiation damage on the crystal, measurements were performed in a short amount of time, i.e. less than a minute. As a result, a low completeness was obtained.

### Structure determination from single-crystal diffraction data

### Co-crystal !!

The proposed indexation by dials is as follows:
- Space group: P 2/m (n°10)
- Unit cell: 15.4338, 18.78041, 21.8797, 90.0, 95.3091, 90.0

A total of five single crystals were mounted and measured, then their diffraction reflections were integrated. Out of these 5 measurements, the best integration result led to an overall measure of quality of Rmerge = 3.74%. (range=1-4%)

The structure was solved using the shelXT program (Sheldrick, G.M. 2015, Acta Cryst. A71, 3-8) with the olex2 GUI interface (Dolomanov, O.V. et al, 2009, J. Appl. Cryst. 42, 339-341). The structure was then refined using shelXL (Sheldrick, G.M., 2015, Acta Cryst. C71, 3-8), the R-factors are R1= 6.01% (good quality <5%, accepted if <10%), wR2= 18.00% (<15%), GooF= 1.59 (~1).

After refinement, the space group and cell parameters are as follows:
- Space group: P 21/n (n°14)
- Unit cell (Å): 15.4338(2), 18.7804(2), 21.8797(2), 90.0, 95.3090(10), 90.0
- Volume (Å3): 6314.69(12)

The asymmetric unit contains two independent molecules of Pitolisant HCl and four independent molecules of 4-hydroxybenzoic acid.

### Co-crystal I

The proposed indexation by dials is as follows:
- Space group: P 2/m (n°10)
- Unit cell: 17.2543, 30.6731, 18.5548, 90, 97.1331, 90

A total of 5 single crystals were mounted and measured, then their diffraction reflections were integrated. Out of these 5 measurements, the best integration result led to an overall measure of quality of Rmerge = 5.97%. (range=1-4%)

The structure was solved using the shelXT program with the olex2 GUI interface. The structure was then refined using shelXL, the R-factors are R1= 8.07% (good quality <5%, accepted if <10%), wR2= 24.05% (<15%), GooF= 1.379 (~1).

After refinement, the space group and cell parameters are as follows:
- Space group: P 21/n (n°14)
- Unit cell (Å): 17.2543(5), 30.6731(6), 18.5548(3), 90.0, 97.133(2), 90.0
- Volume (Å3): 9744.0(4)

The asymmetric unit contains three independent molecules Pitolisant HCl, six independent molecules of 4-aminobenzoic acid and half a water molecule.

In conclusion, the structure of two co-crystals were solved successfully.

Both samples crystallize in the P21/n space group. Co-crystal II exhibits a volume of 6314.69(12) Å³ with two Pitolisant HCl molecules and four 4-hydroxybenzoic acid molecules in the asymmetric unit, while sample Co-crystal I exhibits a volume of 9744.0(4)Å³ with half a water molecule, three Pitolisant HCl molecules and six 4-aminobenzoic acid molecules in the asymmetric unit cell. Pitolisant HCl in Co-crystal II shows a statistical disorder along the C-O-C chains, which was modeled with two different configurations. Sample Co-crystal I also show disorder at the same position but to a lower extend.

### Example 3: Diffraction pattern resulting from the structure resolution

### Co-crystal I

The crystal structure resolved for Co-crystal I is used to simulate the X-ray powder diffraction patterns with Mercury software (C. F. Macrae et al. 2006, J. Appl. Cryst., 39, 453-457).

This simulated pattern is compared with the experimental pattern in Figure 1B. Both patterns are quite similar.

### Co-crystal II

The crystal structure resolved for Co-crystal II is used to simulate the X-ray powder diffraction patterns with Mercury software. This simulated pattern is compared with the experimental pattern in Figure 2B. Both patterns are quite similar.

### EQUIVALENTS

Those skilled in the art will recognize or be able to ascertain using no more than routine experimentation many equivalents to the specific embodiments disclosed herein. Those of ordinary skill in the art will appreciate that various changes or modifications to this description may be made without departing from the spirit or scope of the present disclosure, as defined in the following claims.

## Claims

1. A co-crystal of pitolisant comprising pitolisant hydrochloride represented by Formula (I): 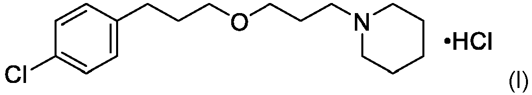 and a benzoic acid derivative of formula (II): 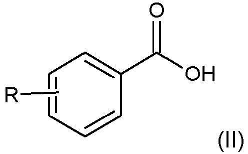 as a coformer,
Where in formula (II) R is located on any available position on position 2, 3 or 4 of the phenyl ring with respect to the carboxyl group, and R is chosen from the group consisting of NH₂, OH, NO₂, Halogen atoms and CN, and the solvated or anhydrous forms thereof.

2. The co-crystal according to claim 1, wherein in formula (II), R is located on the 4-position of the phenyl ring.

3. The co-crystal according to claim 1 or 2 wherein in formula (II), R is chosen from NH₂ and OH.

4. The co-crystal according to any of the preceding claims wherein the coformer of formula (II) is chosen from 4-aminobenzoic acid and 4-hydroxybenzoic acid.

5. The co-crystal according to any of the preceding claims comprising pitolisant HCl and 4-aminobenzoic acid, and optional water.

6. The co-crystal according to claim 5 wherein said co-crystal is **characterized by** having an X-ray diffraction pattern comprising at least one of the following peaks, in terms of 2-theta (2θ), at 10.88°, 13.21°, 14.97°, 15.81°, 17.08°, 17.32°, 17.99°, 18.56°, 18.91°, 20.48°, 22.49°, 25.85° (± 0.2°), or any of the combinations thereof.

7. The co-crystal according to claim 5 or 6, **characterized by** having an X-ray diffraction pattern substantially as shown in pattern 2 in FIG. 1B.

8. The co-crystal according to any of claims 5 to 7, having an asymmetric unit comprising three molecules of pitolisant HCl, six molecules of 4-aminobenzoic acid and half a water molecule.

9. The co-crystal according to any of claims 1 to 4 comprising pitolisant HCl and 4-hydroxybenzoic acid.

10. The co-crystal according to claim 10 wherein said co-crystal is **characterized by** having an X-ray diffraction pattern comprising at least one of the following peaks, in terms of 2-theta (2θ), at 6.21°, 8.11°, 9.41°, 12.75°, 13.41°, 14.71°, 14.88°, 16.94°, 18.82°, 24.29°, 25.26°, 25.71° (± 0.2°), or any of the combinations thereof.

11. The co-crystal according to claim 9 or 10, **characterized by** having an X-ray diffraction pattern substantially as shown in pattern 2 in FIG. 2B.

12. The co-crystal according to any of claims 10 to 11, having an asymmetric unit comprising two molecules of pitolisant HCl and four molecules of 4-hydroxybenzoic acid.

13. The co-crystal according to claim 5 wherein said co-crystal is **characterized by** having an X-ray diffraction pattern comprising at least one of the following peaks, in terms of 2-theta (2θ), at 9.85°, 10.69°, 12.79°, 17.03°, 17.58°, 17.91°, 18.44°, 18.92°, 19.25°, 22.11°, 22.48°, 27.99° (± 0.2°).

14. The co-crystal according to claim 12 or 13, **characterized by** having an X-ray diffraction pattern substantially as shown in pattern 6 in FIG. 3.

15. A process of preparation of the co-crystal according to any of the preceding claims comprising mixing pitolisant hydrochloride and said coformer in an organic solvent.

16. A pharmaceutical composition comprising the co-crystal according to any of the claims 1 to 14, and optionally a pharmaceutically acceptable excipient.

17. The pharmaceutical composition according to claim 16, wherein the pharmaceutically acceptable excipient is selected from the group consisting of colloidal silicon dioxide, crospovidone, magnesium stearate, microcrystalline cellulose, polyethylene glycol, polyvinyl alcohol, talc, and titanium dioxide.

18. A dosage form comprising a co-crystal, or pharmaceutical composition of any of claims 1 to 14.

19. The dosage form of claim 18, wherein the dosage form is a tablet, caplet, or capsule.

20. A co-crystal, pharmaceutical composition, or dosage form of any of claims 1 to 14, for use in the treatment of a sleep disease or disorder.

21. The co-crystal, pharmaceutical composition, or dosage form for use of claim 20, wherein the sleep disease or disorder is chosen from excessive daytime sleepiness (EDS), cataplexy, narcolepsy, sleep apnea (e.g., obstructive sleep apnea), sleep induced apnea, and diurnal somnolence, severe fatigue and idiopathic hypersomnia.

22. The co-crystal, pharmaceutical composition, or dosage form for use of claim 20 or 21, wherein the disease or disorder is in a subject with narcolepsy.

23. The co-crystal, pharmaceutical composition, or dosage form for use of any of claims 20 to 21, wherein the disease or disorder is excessive daytime sleepiness (EDS).

24. The co-crystal, pharmaceutical composition, or dosage form for use of any of claims 20 to 21, wherein the disease or disorder is cataplexy.
